Europäisches Patentamt

European Patent Office

Office européen des brevets

(19)

(11)     EP 0 778 783 B1

(12)     **FASCICULE DE BREVET EUROPEEN**

(45) Date de publication et mention
de la délivrance du brevet:
**22.09.2004   Bulletin 2004/39**

(21) Numéro de dépôt: **96918797.0**

(22) Date de dépôt: **03.07.1996**

(51) Int Cl.[7]: **A61M 1/16**

(86) Numéro de dépôt international:
**PCT/IB1996/000644**

(87) Numéro de publication internationale:
**WO 1997/002057 (23.01.1997 Gazette 1997/05)**

(54) **APPAREIL DE DIALYSE AUTOMATIQUE**

GERÄT FÜR DIE AUTOMATISCHE HÄMODIALYSE

AUTOMATIC DIALYSIS APPARATUS

(84) Etats contractants désignés:
**AT BE CH DE ES FR GB IT LI NL SE**

(30) Priorité: **04.07.1995   IT   TO950560**

(43) Date de publication de la demande:
**18.06.1997   Bulletin 1997/25**

(73) Titulaire: **GAMBRO HOSPAL (Schweiz) AG
4001 Basel (CH)**

(72) Inventeurs:
• **BOSETTO, Antonio
I-41036 Mirandola (IT)**

• **PAOLINI, Francesco
I-41100 Modena (IT)**

(74) Mandataire: **Sutto, Luca et al
Gambro Intellectual Property Department
Hospal International Marketing Management
61, avenue Tony Garnier
69007 Lyon (FR)**

(56) Documents cités:
**EP-A- 0 186 973          EP-A- 0 291 421
WO-A-93/00938          DE-A- 2 734 075
GB-A- 2 052 303**

**Description**

La présente invention concerne un appareil de dialyse automatique.

Comme il est connu chez les malades soumis à un traitement hémodialytique classique, on voit souvent apparaître des effets collatéraux caractérisés par de légers phénomènes hypotensifs ou même par des phénomènes de collapsus.

Ces phénomènes sont provoqués par le fait que les appareils actuels pour dialyse prévoient pour leur fonctionnement, l'établissement d'un ensemble de valeurs prédéfinies et agissent sur le malade en chaîne ouverte, n'utilisant aucune information de retour provenant du malade lui-même. Ceci conduit à avoir dans un certain pourcentage des cas, des complications cliniques provoquées par l'extrême variabilité de la capacité du malade à supporter une soustraction pondérale donnée.

Pour obvier à ces inconvénients désagréables, il convient de mettre sous contrôle certains paramètres du malade indicatifs de son état de santé.

Les inventions précédentes, par exemple, concernent des appareils se fondant sur le contrôle de la volémie du malade, mesurée comme variation relative du volume du sang (BV) circulant dans le corps par rapport au début de la séance, et des systèmes de contrôle aptes à faire en sorte que le BV suive une trajectoire prédéfinie, dans le but de résoudre le problème de l'hypovolémie qui se manifeste souvent de manière improvisée. La mesure de la variation en pourcentage du volume du sang circulant dans le corps peut être exécutée par plusieurs procédés comme, par exemple, celui qui est décrit dans le brevet TO91A000527 au nom de la demanderesse, qui prend en considération comme grandeur indicative du bien être du malade la pression artérielle systolique, dont la corrélation avec le BV est connue.

D'autres inventions, en revanche, concernent les appareils qui contrôlent des paramètres du malade particulier, comme il est dit par exemple dans les demandes de brevet EP-A-029 793 et EP-A-089 003. En particulier, le brevet EP-A-029 793 au nom de Thomasset décrit un système comprenant une mesure de l'impédance du sang et un appareil qui injecte du chlorure de sodium au malade lorsque la valeur d'impédance mesurée sort de seuils prédéfinis. Ce système réalise une simple rétroaction de type marche-arrêt à peine efficace du point de vue clinique.

Le brevet EP-A-089 003 au nom de TOYOTA CHUO KENKYUSHO décrit en revanche un appareil fondé sur un système de contrôle élémentaire visant à faire en sorte que le volume de sang suive une trajectoire préfixée au début du traitement, se fondant sur l'état du malade. Dans ce système connu, pour obtenir les variations du volume du sang, on mesure l'hématocrite. Toutefois, il est démontré que la mesure de l'hématocrite ne suffit pas pour déterminer les variations du volume du sang, dans la mesure où l'hypothèse sur laquelle se fonde la relation biunivoque entre l'hématocrite et la variation du volume du sang, c'est-à-dire l'hypothèse de constance du volume cellulaire total, est rarement vérifiée. En tous cas, le type de contrôle proposé s'adapte mal à l'application à une population entière de malades ou à des séances diverses pour un seul malade, ou même à divers moments de la même séance, en ce qu'il ne tient pas compte des réactions individuelles au traitement, qui non seulement sont généralement différentes d'un malade à l'autre mais peuvent également varier chez le même individu à divers moments. En outre, dans ce brevet, il n'est pas tenu compte des limitations auxquelles est soumise la manipulation des paramètres de la machine. C'est pourquoi le système décrit est peu efficace dans les cas où des variations significatives du comportement intradialytique du malade se produisent.

A l'heure actuelle un aspect non résolu dans aucun système de dialyse existant est l'harmonisation complète d'objectifs cliniques différents, dont l'obtention porte continuellement à la nécessité d'effectuer des opérations de compromis entre l'obtention ponctuelle d'un résultat, par exemple, la baisse du poids final (TWL) qui contribue au maintien des conditions de bien-être du malade à moyen et à long terme, et la réduction finale du volume du sang (TBV) qui permet la stabilisation hémodynamique intradialytique avec une réduction conséquente des pathologies en cours de traitement. Dans les dialyses traditionnelles, l'évaluation clinique relative à ce compromis est effectuée de manière approximative par le médecin, qui, dialyse après dialyse, choisit les paramètres de traitement qu'il retient, sur la base de son expérience propre, après en avoir déterminé les effets sur le malade, par exemple, la vitesse de baisse de poids (WLR) définie comme la différence entre la vitesse d'ultrafiltration (UFR) et la vitesse de transfusion ($Q_{inf}$), la conductivité du liquide de dialyse (CD), et éventuellement est contraint à revoir son choix au cours du traitement à la suite de phénomènes de collapsus.

Dans le système de dialyse automatique mentionné, il faut toujours opérer ce compromis manuellement en phase de prescription du traitement, et revoir la situation au cours du traitement en fonction de l'impossibilité d'obtenir simultanément les objectifs ponctuels atteints. Cette gestion discontinue du traitement dialytique aboutit à rendre partiellement vains les avantages obtenus par le système automatique. En fait, l'exécution des dialyses automatiques selon les modalités mentionnées nécessite une grande expérience dans la formalisation de la prescription sous la forme des trajectoires désirées, ainsi que de la rapidité d'intervention au cas où la prescription serait inappropriée ou ne pourrait être poursuivie du fait des conditions contingentes du malade.

Le but de la présente invention est donc de réaliser un procédé et un appareil de dialyse automatique exempts des inconvénients décrits ci-dessus, qui permettent donc de formaliser la prescription du médecin aussi bien en termes de WL qu'en termes de BV, en définissant en outre l'importance relative de l'obtention de chacun des deux objectifs finaux considérés isolement au moyen de limites de tolérance accordées à chacun d'entre eux.

En particulier, on cherche à réaliser un procédé et un appareil en mesure d'obtenir la réalisation d'un état final du malade entre les limites prévues de la prescription clinique qui au début du traitement permet de définir des intervalles de valeurs variables dans le temps entre lesquelles les paramètres contrôlés BV et WL doivent être comprises à chaque instant du traitement.

Selon la présente invention, on réalise un appareil de dialyse automatique tels que défini par la revendication 1.

Pour mieux comprendre l'invention, on décrira maintenant une forme de réalisation préférée, à titre d'exemple non limitatif en se référant aux dessins annexés, sur lesquels:

- la figure 1 présente un organigramme relatif à l'algorithme général utilisé dans le procédé de dialyse objet de l'invention;
- les figures 2 et 3 présentent deux organigrammes détaillés relatifs à l'organigramme de la figure 1;
- la figure 4 présente un schéma fonctionnel de l'appareil objet de l'invention et,
- les figures 5 à 7 montrent des diagrammes relatifs à l'évolution des variables utilisées dans le procédé de l'invention.

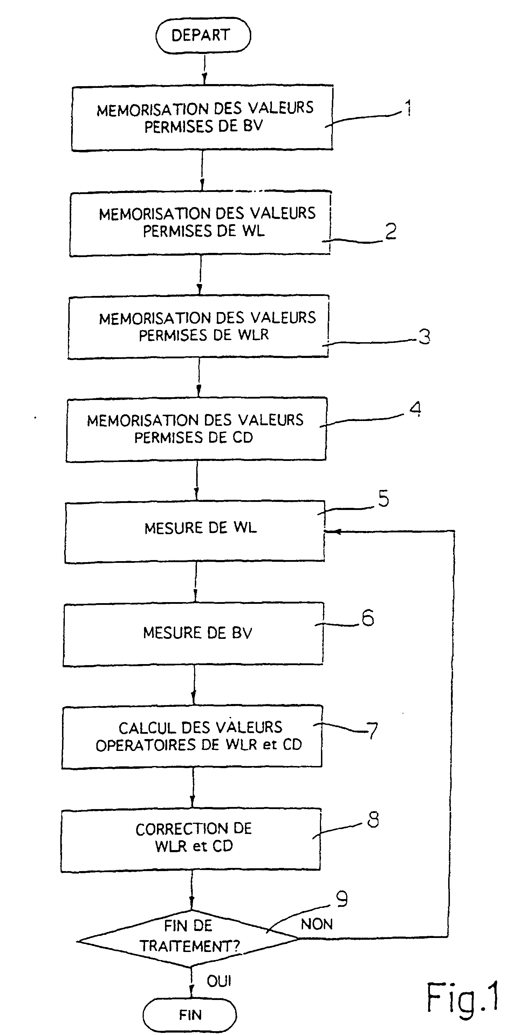

Sur la figure 1 est illustré un schéma fonctionnel qui montre les phases relatives à une séance de dialyse. En détail, la séance commence par la mémorisation des intervalles de variations permis par les quelques variables contrôlées, au cours du traitement, avec la valeur minimale et la valeur maximale permises. En particulier, on mémorise les intervalles de variations permis pour la réduction du volume du sang BV (bloc 1), pour la baisse du poids WL (bloc 2), pour la vitesse de baisse du poids WLR (bloc 3) et pour la conductivité du liquide de dialyse CD (bloc 4). Ensuite, on commence le traitement dialytique proprement dit et on effectue les mesures de WL (bloc 5) et de BV (bloc 6). Ces paramètres malade étant notés, on calcule les valeurs appropriées de WLR et de CD (bloc 7), et celles-ci sont utilisées pour corriger les valeurs précédentes de CD et de la vitesse de baisse de poids (bloc 8). Afin que le traitement n'arrive pas à la fin (comme on le vérifie au moyen du bloc 9) on répète les étapes comprises entre le bloc 5 et le bloc 8.

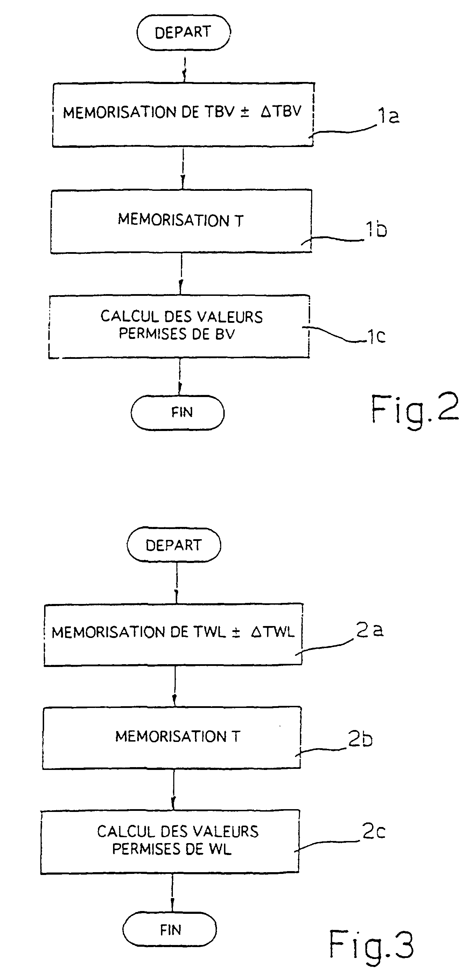

Les phases représentées par les blocs 1 et 2 comprennent à leur tour les séquences de passage représentées à la figure 2, et, respectivement 3. En fait, comme il est montré sur la figure 2, la mémorisation de l'intervalle de variation permis de BV (bloc 1) comprend la mémorisation de la valeur de BV voulue à la fin du traitement (TBV) et les tolérances relatives ($\pm\Delta$TBV) (bloc 1a), la mémorisation de la durée du traitement (T) (bloc 1b) et le calcul des extrêmes de l'intervalle de variation de BV permis au cours du traitement (bloc 1c) selon un modèle mathématique précis ou selon des évolutions valables pour tous les malades dérivées de l'analyse des données cliniques.

En revanche, la mémorisation de WL (bloc 2) comprend, figure 3, la mémorisation de la valeur de WL voulue à la fin du traitement (TWL) et des tolérances relatives ($\pm\Delta$TWL) (bloc 2a), la mémorisation de la durée du traitement (T) et des tolérances relatives ($\pm\Delta$T) (bloc 2b) et le calcul des extrêmes de l'intervalle de variation de WL permis au cours du traitement (bloc 2) selon un modèle mathématique précis ou selon des évolutions valables pour tous les malades dérivées de l'analyse de données cliniques.

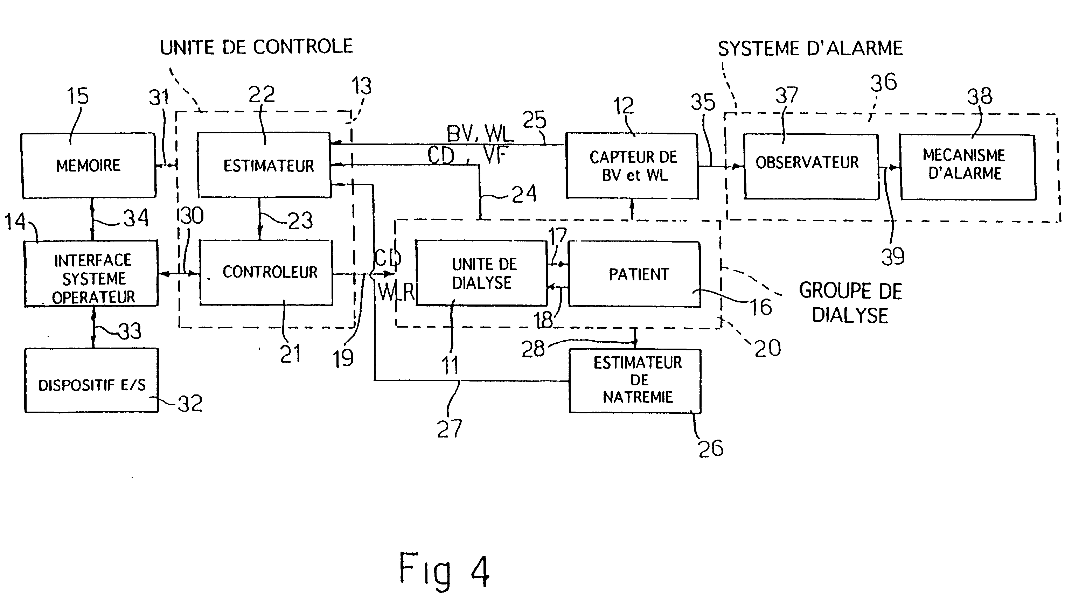

Le procédé de dialyse automatique décrit ci-dessus est réalisé par l'appareil 10, dont le schéma fonctionnel est illustré sur la figure 4. L'appareil 10 comprend une unité de dialyse 11 recevant comme information prélable deux paramètres machine (WLR et CD), un groupe de capteurs 12 pour la mesure de deux paramètres malade effectifs (BV et WL) que l'on veut contrôler, une unité de contrôle 13 du type adaptatif qui, fonctionnant sur les paramètres machine WLR et CD que celle-ci fournit à l'unité de dialyse 1 1 et sur les paramètres malades effectifs BV et WL mesurés pour le groupe des capteurs 12, fait prendre aux paramètres malades BV et WL les évolutions désirées. Ces évolutions sont fixées préalablement par un opérateur au moyen de dispositifs d'entrée/sortie 32 qui coopèrent avec une mémoire 1 5 par l'intermédiaire d'une interface appareil/opérateur 14. De façon connue, l'unité de dialyse 11, de type traditionnel, est reliée au système vasculaire du malade, indiqué schématiquement sur la figure 4 par le bloc 16, au moyen d'un couple de lignes 17, 18, respectivement sortant de l'unité de dialyse 11 et y entrant, qui se joignent à un filtre dans lequel on fait s'écouler le sang du malade. Du point de vue du contrôle adaptatif réalisé par le moyen de l'unité de contrôle 13, l'unité de dialyse 11 et le malade 1 6 forment un groupe de dialyse 20 dont la liaison avec le groupe de capteurs 12 est représentée schématiquement par une flèche.

En détail, l'unité de contrôle 13 est reliée à l'unité de dialyse 11 par le moyen d'une ligne 19 qui comprend un contrôleur 21 et un estimateur 22 communiquant avec le contrôleur 21 par l'intermédiaire de la ligne 23; le contrôleur 21 et l'estimateur 22 définissent ensemble un contrôleur adaptatif et sont représentés séparément uniquement à titre illustratif, mais en général sont réalisés par un seul composant. L'estimateur 22 reçoit à l'entrée des signaux provenant du groupe de dialyse 20 par l'intermédiaire de la ligne 24, du groupe de capteurs 12 par l'intermédiaire d'une ligne 25 et d'un estimateur de natrémie 26 par l'intermédiaire d'une ligne 27. L'estimateur de natrémie 26 est, en outre, relié au groupe de dialyse 20 par l'intermédiaire de la ligne 28.

L'unité de contrôle 13 est, en outre, reliée par l'intermédiaire d'une ligne électrique 30, à l'interface 14 pour la lecture des valeurs désirées des paramètres malades BV et WL, de WLR, de CD, de TWL $\pm$ $\Delta$TWL, de TBV $\pm$ $\Delta$ TBV et de T. L'unité de contrôle 13, en outre, communique, par l'intermédiaire d'une ligne 31, avec la mémoire 15 pour l'échange des données nécessaires à son élaboration et des résultats des élaborations elles-mêmes. Les deux lignes 30, 31 sont représentées séparément dans un but illustratif uniquement; de fait, elles peuvent être formées par une liaison unique.

L'interface 14 est reliée avec le dispositif d'entrée/sortie 32 à travers une ligne 33 et avec la mémoire 1 5 par l'intermédiaire d'une ligne 34.

Enfin le groupe de capteurs 12, communique, par l'intermédiaire d'une ligne 35, avec un système d'alarme 36 comprenant un observateur 37 en mesure de relever une impossibilité, de la part de l'unité de contrôle 13, d'effectuer sa fonction de maintien des variables contrôlées BV et WL entre les limites d'oscillation stabilisée, et d'un mécanisme d'alarme 38, relié à l'observateur 37 par l'intermédiaire d'une ligne 39 et en mesure d'avertir l'opérateur de cette impossibilité de contrôle.

Tout bloc dont est composé l'appareil 10 de l'invention est à son tour réalisé en circuit au moyen de techniques en soi connues. Par exemple, diverses techniques de contrôle connues peuvent être utilisées pour obtenir le résultat annoncé. La technique décrite ci-dessous représente une des possibilités choisies et est présentée en titre d'exemple, sans pour autant limiter la généralité des solutions applicables.

L'unité de contrôle 13 se fonde sur l'évaluation d'une fonction d'erreur qui est maintenue à une valeur minimum au cours de la dialyse. Dans la réalisation effectuée la fonction erreur est la combinaison linéaire des intervalles des paramètres malade d'une fois sur l'autre mesurées à partir de points médians des intervalles admis; dans cette combinaison linéaire ou utilise des pondérations de valeur inversement proportionnelles à l'amplitude des intervalles euxmêmes. En particulier la fonction d'erreur considérée est:

$$err(BV(t),WL(t))= K_{BV}.err_{BV} + K_{WL}.err_{WL}$$

où:

$$err_{BV} = BV(t) - \frac{BV_{min}(t) + BV_{max}(t)}{2}$$

$$err_{WL} = WL(t) - \frac{WL_{min}(t) + WL_{max}(t)}{2}$$

$$K_{BV} = (BV_{min}(t) - BV_{max}(t))^{-1}$$

$$KWL = (WL_{min}(t) - WL_{max}(t))^{-1}$$

où $K_{BV}$ et $K_{WL}$ sont des facteurs de normalisation (fonction de l'importance relative de l'obtention de chacun des deux objectifs finals et établis implicitement par le médecin au moyen de la définition des valeurs admissibles).

D'autres fonctions d'erreur sont possibles, dans ce même but de maintenir des variables de contrôle dans l'intervalle des valeurs admises. Il est également possible d'utiliser des fonctions d'erreur différentes pour chacune des valeurs machine WLR et CD, qui, du point de vue du contrôle, représentent deux branches de réalisation.

A la base du résultat de la fonction erreur (fonction relative à chaque grandeur), les valeurs courantes de WLR et CD sont modifiées au moyen d'un régulateur de type classique. Dans la réalisation effectuée, on a recours à un algorithme de contrôle numérique programmé sur un calculateur ou équivalent, dans les limites des systèmes continus, à une correction sur la branche d'aller avec réseau à assiette, dont la fonction de transfert, dans le domaine des transformées de Laplace, présente un transfert de gain HO, deux pôles $p_1$, $p_2$ et deux zéros $z_1$, $z_2$. Les pôles et les zéros ainsi que le gain déterminent le comportement dynamique du système de contrôle et leur choix détermine la stabilité du contrôle et la vitesse et la précision d'obtention du résultat.

Les valeurs opportunes des paramètres du réseau correcteur de chaque branche de réalisation (WLR et CD) pourraient être stabilisées à priori au moyen des techniques de contrôle habituelles fondées sur l'évaluation de la réponse dynamique du paramètre malade BV(t) dans des dialyses réelles, au moyen de la formulation de modèles mathématiques équivalents au malade d'après les données de lecture. Toutefois, cette approche n'est souvent pas adéquate, car elle présuppose une réaction individuelle constante à WLR et à CD, considérée comme les déclencheurs en mesure de modifier le BV (outre le WL), tandis qu'en réalité cette réaction est susceptible de variations d'un individu à l'autre et chez le même individu à des moments différents.

Dans le but d'adapter le comportement propre aux variations individuelles des malades, le contrôleur réalisé utilise donc un module, représenté par l'estimateur 22, qui permet d'identifier les diverses réponses du malade aux sollicitations de l'appareil de dialyse, avec réalisation périodique d'une estimation des paramètres du modèle unité de dialyse-malade liés à la sensibilité du BV aux variations de WLR et CD. La disponibilité du modèle unité de dialyse-malade, identifiée au temps t, permet de calculer, au moyen de technique de contrôle optimal, les paramètres du réseau de correction décrit (WLR et CD) ou d'effectuer la synthèse du réseau de correction le plus opportun pour parvenir dans le plus court temps possible et avec une marge de stabilité suffisante à la valeur minimum de la fonction erreur.

En ce qui concerne en revanche l'introduction initiale des paramètres désirés, une réalisation possible simplifiée du procédé pour programmer les valeurs de limite de tolérance en fonction du temps est ensuite représentée, comme il est dit plus en détail ci-dessous, par l'introduction des valeurs limites aux fins de dialyse $BV_{min}(t_{fin})$, $BV_{max}(t_{fin})$, $WL_{min}(t_{fin})$, $WL_{max}(t_{fin})$, tandis que la morphologie de la courbe de raccord de ces points finals aux valeurs initiales $BV_{min}(O)$, $BV_{max}(O)$, $WL_{min}(O)$, $WL_{max}(O)$ (par construction nulles, dans la mesure où il n'y a pas de variation du volume du sang, ni baisse de poids au début du traitement) est dérivée de l'expérience et programmée sous la forme d'une courbe moyenne normalisée valable pour tous les malades, ou d'une loi mathématique d'interpolation définie à priori ou encore d'un modèle mathématique qui décrit l'entité des transferts hydriques qui se produisent chez le malade en fonction de la perte de poids et des échanges par diffusion à travers la membrane de dialyse.

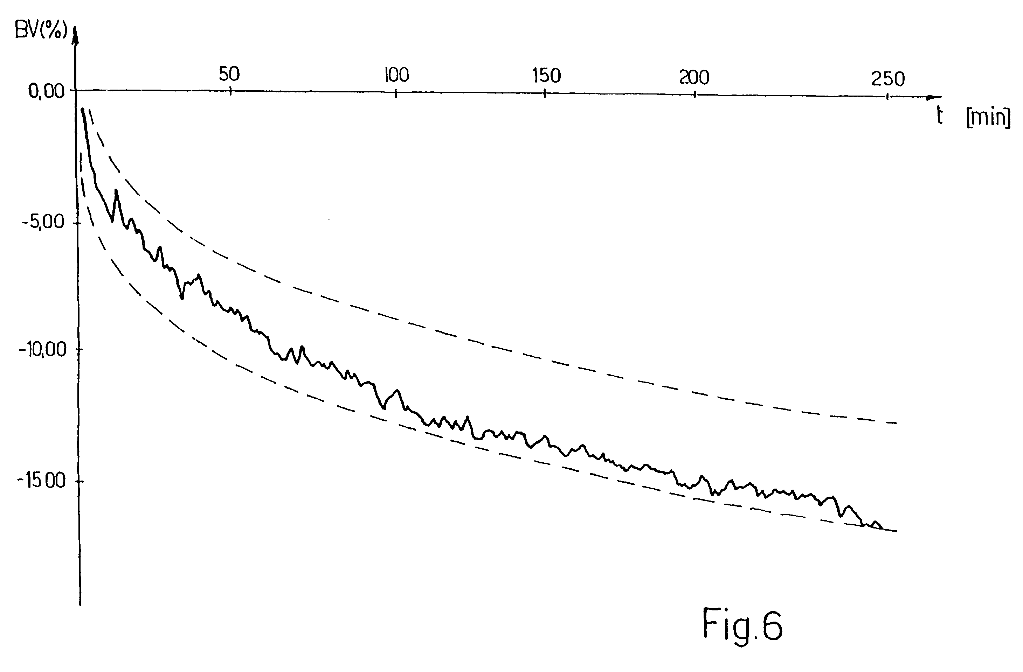

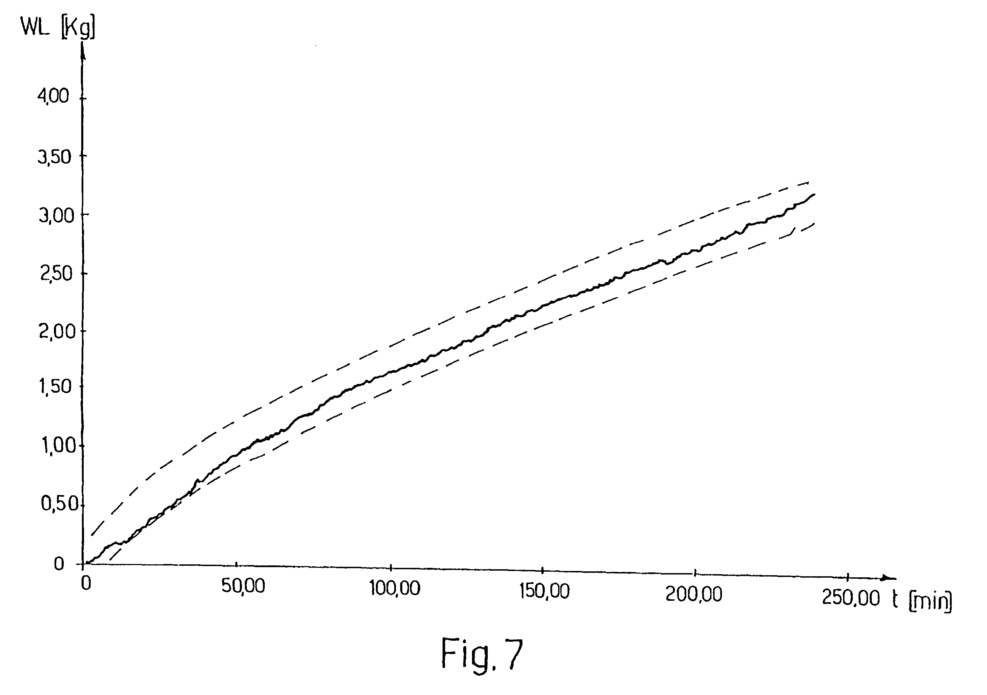

Dans des essais effectués par la demanderesse, on a recours à des courbes empiriques obtenues au moyen de la réponse au traitement d'une population de malades qui n'ont pas présenté de symptomatologie intradialytique. Sur la figure 6 est en fait illustrée l'évolution de la variation du volume du sang au cours du traitement (ligne continue) et les marges relatives de variation permise (lignes en pointillé), tandis que sur la figure 7 est illustrée l'évolution de la baisse du poids total au cours du traitement (ligne continue) et les marges relatives de variation permise (lignes en pointillé). Il est, en outre, utile de se rappeler que la valeur de la baisse de poids total est égale à l'intégrale de la vitesse de la baisse de poids.

La variabilité des marges accordée dans l'obtention des objectifs est dépendante de la variabilité de la population de patients hémodialysés qui comporte des individus particulièrement sensibles aux variations du volume de sang et par conséquent sujets à des pathologies intradialytiques à la charge du système cardiovasculaire, tandis qu'il existe des malades plus tolérants, mais qui ont cependant besoin d'un contrôle de poids corporel rigoureux.

En ce qui concerne en revanche l'observateur 37 pour la tendance des variables contrôlées vis-à-vis des situations de franchissement des limites imposées, il a pour but de prédire un futur franchissement et d'en aviser l'opérateur avec une avance opportune afin qu'il puisse mettre en oeuvre, à temps, des manoeuvres correctives comme celles décrites ci-dessus. Les techniques de réalisation peuvent, par exemple, se fonder sur la mesure des dérivées par rapport au temps des différences $BV(t)-BV_{min}(t)$, $BV(t)-BV_{max}(t)$, $WL(t)-WL_{min}(t)$, $WL(t)-WL_{max}(t)$.

L'estimateur de natrémie 26 se fonde en revanche sur le calcul de la natrémie du malade $Na(t)$ au moyen de l'intégration de l'effet de CD dans un modèle unité de dialyse-malade à partir d'une valeur mesurée ou présumée de la concentration initiale de sodium dans l'eau plasmatique du malade. Selon une première solution, il est possible d'avoir recours à une simple représentation monocompartimentale du volume de distribution du sodium chez le malade, dans lequel les échanges sont représentés par la diffusion et la convection au travers de la membrane de dialyse.

On peut également avoir recours à des moyens de mesure de la concentration ionique plasmatique, lesquels réalisent une mesure directe ou indirecte de $Na(t)$ et permettent ainsi d'éviter d'avoir recours à des modèles mathématiques imprécis. En disposant de l'information sur l'évolution de la natrémie plasmatique du malade, on peut ensuite en tenir compte dans la fonction qui pilote le dispositif d'actionnement CD. Par exemple, la précédente fonction d'erreur peut devenir:

$$err(BV(t),WL(t))= K_{BV1}.err_{BV} + K_{WL}.err_{WL} + K_{Na}.err_{Na}$$

dans laquelle les termes communs à la fonction d'erreur précédente prennent les mêmes valeurs et expressions, tandis que :

$$err_{Na}= Na(t) - \frac{Na_{min}(t) + Na_{max}(t)}{2}$$

$$K_{Na} = (Na_{min}(t) - Na_{max}(t))^{-1}$$

avec un facteur de normalisation $K_3$ semblable au précédent.

Le fonctionnement de l'appareil pour dialyse automatique 10 est décrit en référence aux organigrammes des figures 1-3.

Pour obtenir le résultat du contrôle, l'appareil 10 vérifie au temps t l'appartenance des valeurs mesurées de BV et de WL aux intervalles de valeurs admis et dispose de la réalisation de WLR et CD à chaque instant de temps entre les intervalles de valeurs admissibles et afin de les maintenir simultanément éloignées des limites de variabilité admise pour les variables contrôlées de WL et de BV.

En particulier, le traitement de dialyse commence par l'introduction, par l'opérateur, des intervalles de variation permis au cours du traitement (bloc 1 ) de BV, de WL, de WLR et de CD ainsi que de la durée T du traitement. Ces valeurs sont introduites par l'intermédiaire des dispositifs E/S 32 et successivement gérées par l'interface 14; ceiie-ci les mémorise dans la mémoire 15 et les envoie à l'unité de contrôle 13. L'unité de contrôle 13 commande, ensuite, le groupe de dialyse 20 sur la base des paramètres machine initiaux WLR et CD et ainsi commence la séance proprement dite. Le groupe de capteurs 12 relève les premiers échantillons des paramètres malade BV et WL (blocs 5, 6) et les envoie, avec les paramètres machine CD, WLR et la natrémie calculée par l'estimateur de natrémie 26, à l'estimateur 22 des paramètres du modèle unité de dialyse-malade. L'estimateur 22 calcule ainsi les valeurs de coefficients de contrôle 21 qui lient les WLR et CD effectifs aux erreurs sur BV et WL et (bloc 7) les communiquent au contrôleur 21, lequel corrige les paramètres machine CD et WLR envoyés au groupe de dialyse 20 (bloc 8).

L'observateur 37 relève les variations des paramètres malade BV et WL et communique au mécanisme d'alarme 38 une éventuelle tendance des paramètres BV et WL à dépasser les intervalles permis, de manière que l'opérateur puisse décider de continuer la dialyse automatique ou d'intervenir sur les limites de variation admises. Si cela n'était pas possible l'opérateur pourrait également décider d'interrompre le traitement automatique et conclure la séance de façon manuelle.

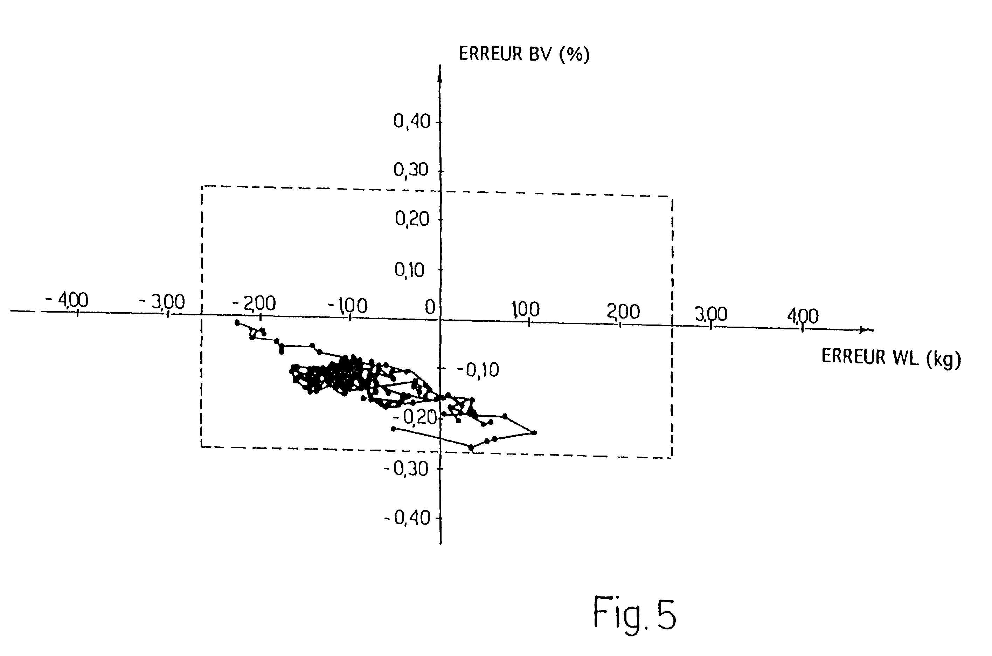

Les dispositifs E/S 32 permettent, outre l'introduction des données utiles à l'appareil pour agir correctement, la représentation synthétique (figure 5) du positionnement simultané des paramètres malade par rapport aux intervalles d'admissibilité courants, de manière que le responsable du traitement de dialyse puisse établir en un coup d'oeil la conduite correcte du traitement de dialyse automatique et l'utilisation des marges de variation admises.

Le cycle d'acquisition des paramètres machine et malade et le calcul des nouveaux paramètres machine continuent jusqu'à ce que se soit écoulé le temps T imposé comme durée de la séance.

Il est enfin clair que l'on peut apporter au procédé et à l'appareil ici décrit et représenté des modifications et variantes sans pour autant sortir du cadre de l'invention selon les revendications. Par exemple, il est possible de réaliser un appareil privé d'estimateur de natrémie ou de mécanisme d'alarme sans pour autant altérer ses fonctions.

**Revendications**

1. Appareil de dialyse automatique, comprenant une unité de dialyse, des moyens d'acquisition de valeurs admises de paramètres d'entrée et de valeurs désirées de paramètres malade; des moyens d'acquisition des valeurs effectives desdits paramètres-malade, lesdits paramètres malades comprenant la baisse de poids (WL); des moyens d'acquisition des valeurs effectives des paramètres machine, lesdits paramètres machine comprenant la vitesse de baisse de poids (WLR); et des moyens de contrôle (13) du fonctionnement de l'unité de dialyse au moyen de valeurs opératoires desdits paramètres machine pour faire prendre auxdits paramètres malade lesdites valeurs désirées, **caractérisé en ce que** :

   • les paramètres malade comprennent la variation relative de volume de sang (BV),
   • les paramètres machine comprennent la conductivité du liquide de dialyse (CD),
   • les moyens d'acquisition comprennent:

      - des moyens de mémorisation des valeurs permises de la variation relative de volume de sang en fonction du temps de dialyse;
      - des moyens de mémorisation des valeurs permises de la baisse de poids en fonction du temps de dialyse;
      - des moyens de mémorisation des valeurs permises de la vitesse de baisse de poids en fonction du temps de dialyse;
      - des moyens de mémorisation des valeurs permises de la conductivité du liquide de dialyse en fonction du temps de dialyse;

- les moyens d'acquisition des valeurs effectives desdits paramètres malade comprennent:

  - des moyens d'acquisition (12) des valeurs effectives de la baisse de poids, à chaque instant du temps au cours du traitement;
  - des moyens d'acquisition (12) des valeurs effectives de la variation de volume de sang, à chaque instant du temps au cours du traitement;

- les moyens de contrôle (13) du fonctionnement de l'unité de dialyse comprennent:

  - des moyens de calcul des valeurs opératoires de la vitesse de baisse de poids et de la conductivité du liquide de dialyse selon un modèle mathématigue avant en entrée au moins les paramètres malade effectifs et en sortie la vitesse de baisse de poids et la conductivité du liquide de dialyse;
  - des moyens de correction des valeurs de la vitesse de baisse de poids et de la conductivité du liquide de dialyse, sur la base des valeurs opératoires.

2. Appareil selon la revendication 1, **caractérisé en ce que** les moyens d'acquisition des valeurs admises des paramètres d'entrée et des valeurs désirées des paramètres malade comprennent un dispositif d'introduction et de visualisation de données (32), un dispositif d'interface (14) appareil-opérateur et des dispositifs de mémoire (15).

3. Appareil selon la revendication 1 ou 2, **caractérisé en ce que** les moyens d'acquisition des valeurs effectives des paramètres malade comprennent des moyens capteurs.

4. Appareil selon une des revendications 1 à 3, **caractérisé en ce que** les moyens de contrôle (13) comprennent un contrôleur (21) et un estimateur (22) de la vitesse de baisse de poids et de la conductivité du liquide de dialyse selon ledit modèle mathématique, et **en ce que** l'estimateur (22) comprend des moyens d'optimisation de la vitesse de baisse de poids et de la conductivité du liquide de dialyse, les moyens d'optimisation étant capables d'estimer les coefficients du modèle sur la base d'une évaluation de la variation du volume du sang en fonction de la vitesse de baisse de poids et de la conductivité du liquide de dialyse.

5. Appareil selon une des revendications 1 à 4, **caractérisé en ce qu'**il comprend des moyens d'estimation (26) de la natrémie du malade.

6. Appareil selon les revendications 4 et 5, **caractérisé en ce que** l'estimateur (22) comprend des moyens d'optimisation de la vitesse de baisse de poids et de la conductivité du liquide de dialyse aptes à estimer les coefficients du modèle sur la base d'une évaluation de la variation du volume de sang en fonction de la vitesse de baisse de poids, de la conductivité du liquide de dialyse et de la natrémie.

7. Appareil selon une des revendications 1 à 6, **caractérisé en ce qu'**il comprend des moyens (36) pour relever un dépassement aptes à relever un éventuel dépassement des paramètres malade effectifs à partir des intervalles de valeurs permises.

8. Appareil selon la revendication 7, **caractérisé en ce que** les moyens (36) pour relever le dépassement comprennent des moyens (37) observateurs du dépassement et des moyens (38) pour signaler le dépassement, et **en ce que** l'observateur (37) comprend des moyens de calcul des dérivées par rapport au temps des différences entre les valeurs effectives et les valeurs permises de la variation du volume de sang et entrç les valeurs effectives et les valeurs permises de la baisse de poids.

9. Appareil selon la revendication 2, **caractérisé en ce que** le dispositif d'introduction et de visualisation de données comprend des moyens de représentation synthétique de positions simultanées des variables malade et des intervalles de valeurs permises.

**Patentansprüche**

1. Vorrichtung zur automatischen Dialyse, umfassend eine Dialyseeinheit, Mittel zur Erfassung von zugelassenen Werten von Eingangsparametern und von gewünschten Werten von Patienten-Parametern; Mittel zur Erfassung der tatsächlichen Werte dieser Patienten-Parameter, wobei diese Patienten-Parameter die Gewichtsabnahme (WL) umfassen; Mittel zur Erfassung der tatsächlichen Werte der Maschinen-Parameter, wobei diese Maschinen-Parameter die Geschwindigkeit der Gewichtsabnahme (WLR) umfassen; und Mittel (13) zur Regelung des Betriebs der Dialyseeinheit mit Hilfe von Betriebswerten dieser Maschinen-Parameter, um die Patienten-Parameter diese gewünschten Werte annehmen zu lassen, **dadurch gekennzeichnet, daß**

- die Patienten-Parameter die relative Blutvolumensänderung (BV) umfassen,
- die Maschinen-Parameter die Leitfähigkeit der Dialyseflüssigkeit (CD) umfassen,
- die Erfassungsmittel umfassen:

  - Mittel zur Speicherung der zulässigen Werte der relativen Blutvolumensänderung in Abhängigkeit von der Dialysezeit;
  - Mittel zur Speicherung der zulässigen Werte der Gewichtsabnahme in Abhängigkeit von der Dialysezeit;
  - Mittel zur Speicherung der zulässigen Werte der Geschwindigkeit der Gewichtsabnahme in Abhängigkeit von der Dialysezeit;
  - Mittel zur Speicherung der zulässigen Werte der Leitfähigkeit der Dialyseflüssigkeit in Abhängigkeit von der Dialysezeit;

- die Mittel zur Erfassung der tatsächlichen Werte der Patienten-Parameter umfassen:

  - Mittel (12) zur Erfassung der tatsächlichen Werte der Gewichtsabnahme zu jedem Zeitpunkt während der Behandlung;
  - Mittel (12) zur Erfassung der tatsächlichen Werte der Blutvolumensänderung zu jedem Zeitpunkt während der Behandlung;

- die Mittel (13) zur Regelung des Betriebs der Dialyseeinheit umfassen:

  - Mittel zur Berechnung der Betriebswerte der Geschwindigkeit der Gewichtsabnahme und der Leitfähigkeit der Dialyseflüssigkeit gemäß einem mathematischen Modell, das als Eingang mindestens die tatsächlichen Patienten-Parameter und als Ausgang die Geschwindigkeit der Gewichtsabnahme und die Leitfähigkeit der Dialyseflüssigkeit hat;
  - Mittel zur Korrektur der Werte der Geschwindigkeit der Gewichtsabnahme und der Leitfähigkeit der Dialyseflüssigkeit auf der Grundlage der Betriebswerte.

**2.** Vorrichtung nach Anspruch 1, **dadurch gekennzeichnet, daß** die Mittel zur Erfassung der zugelassenen Werte der Eingangsparameter und der gewünschten Werte der Patienten-Parameter eine Vorrichtung (32) zur Eingabe und Sichtdarstellung von Daten, eine Bediener-Maschine-Schnittstellenvorrichtung (14) und Speichervorrichtungen (15) umfassen.

**3.** Vorrichtung nach Anspruch 1 oder 2, **dadurch gekennzeichnet, daß** die Mittel zur Erfassung der tatsächlichen Werte der Patienten-Parameter Fühlermittel umfassen.

**4.** Vorrichtung nach einem der Ansprüche 1 bis 3, **dadurch gekennzeichnet, daß** die Regelmittel (13) einen Regler (21) und einen Schätzer (22) für die Geschwindigkeit der Gewichtsabnahme und für die Leitfähigkeit der Dialyseflüssigkeit gemäß diesem mathematischen Modell umfassen und daß der Schätzer (22) Mittel zur Optimierung der Geschwindigkeit der Gewichtsabnahme und der Leitfähigkeit der Dialyseflüssigkeit aufweist, die die Koeffizienten des Modells auf der Grundlage einer Bestimmung der Änderung des Blutvolumens in Abhängigkeit von der Geschwindigkeit der Gewichtsabnahme und von der Leitfähigkeit der Dialyseflüssigkeit schätzen können.

**5.** Vorrichtung nach einem der Ansprüche 1 bis 4, **dadurch gekennzeichnet, daß** sie Mittel (26) zum Schätzen der Naträmie des Patienten aufweist.

**6.** Vorrichtung nach den Ansprüchen 4 und 5, **dadurch gekennzeichnet, daß** der Schätzer (22) Mittel zur Optimierung der Geschwindigkeit der Gewichtsabnahme und der Leitfähigkeit der Dialyseflüssigkeit aufweist, die die Koeffizienten des Modells auf der Basis einer Bestimmung der Änderung des Blutvolumens in Abhängigkeit von der Geschwindigkeit der Gewichtsabnahme, von der Leitfähigkeit der Dialyseflüssigkeit und von der Naträmie schätzen können.

**7.** Vorrichtung nach einem der Ansprüche 1 bis 6, **dadurch gekennzeichnet, daß** sie Mittel (36) zum Feststellen eines Überschreitens aufweist, die ein eventuelles Überschreiten der tatsächlichen Patienten-Parameter ausgehend von den Intervallen von zulässigen Werten feststellen können.

8. Vorrichtung nach Anspruch 7, **dadurch gekennzeichnet, daß** die Mittel (36) zum Feststellen des Überschreitens Mittel (37) zur Beobachtung des Überschreitens und Mittel (38) zum Anzeigen des Überschreitens umfassen und daß das Beobachtungsmittel (37) Mittel zur Berechnung der Ableitungen nach der Zeit der Differenzen zwischen den tatsächlichen Werten und den zulässigen Werten der Änderung des Blutvolumens und zwischen den tatsächlichen Werten und den zulässigen Werten der Gewichtsabnahme aufweist.

9. Vorrichtung nach Anspruch 2, **dadurch gekennzeichnet, daß** die Vorrichtung zur Eingabe und zur Sichtdarstellung von Daten Mittel zur synthetischen Darstellung von gleichzeitigen Positionen der Patienten-Variablen und der Intervalle von zulässigen Werten aufweist.

**Claims**

1. Automatic dialysis apparatus, comprising a dialysis unit, means for acquiring allowed values of input parameters and desired values of patient parameters; means for acquiring the actual values of the patient parameters, the said patient parameters comprising the weight loss (WL); means for acquiring the actual values of the machine parameters, the said machine parameters comprising the weight loss ratio (WLR); and means (13) for controlling the operation of the dialysis unit using operating values of the said machine parameters in order to make the said patient parameters take on the said desired values; **characterized in that**:

   - the patient parameters comprise the relative variation in blood volume (BV),
   - the machine parameters comprise the conductivity of the (CD) dialysis fluid,
   - the acquisition means comprise:

     - means for storing the permitted values of the relative variation in blood volume as a function of the dialysis time;
     - means for storing the permitted values of the weight loss as a function of the dialysis time;
     - means for storing the permitted values of the weight loss rate as a function of the dialysis time;
     - means for storing the permitted values of the conductivity of the dialysis fluid as a function of the dialysis time;

   - the means for acquiring the actual values of the said patient parameters comprise:

     - means (12) for acquiring the actual values of the weight loss, at each instant in time during the treatment;
     - means (12) for acquiring the actual values of the variation in blood volume, at each instant in time during the treatment;

   - the means (13) for controlling the operation of the dialysis unit comprise:

     - means for calculating the operating values of the weight loss rate and of the conductivity of the dialysis fluid according to a mathematical model having at least the actual patient parameters as input and the weight loss rate and the conductivity of the dialysis fluid as output;
     - means for correcting the values of the weight loss rate and of the conductivity of the dialysis fluid, on the basis of the operating values.

2. Apparatus according to Claim 1, **characterized in that** the means for acquiring the allowed values of the input parameters and the desired values of the patient parameters comprise a device (32) for inputting and displaying data, an apparatus/operator interface device (14) and memory devices (15).

3. Apparatus according to Claim 1 or 2, **characterized in that** the means for acquiring the actual values of the patient parameters comprise sensor means.

4. Apparatus according to one of Claims 1 to 3, **characterized in that** the control means (13) comprise a controller (21) and an estimator (22) of the weight loss rate and the conductivity of the dialysis fluid according to the said mathematical model, and **in that** the estimator (22) comprises means for optimizing the weight loss rate and the conductivity of the dialysis fluid, the optimization means being capable of estimating the coefficients of the model on the basis of an evaluation of the variation in the blood volume as a function of the weight loss rate and of the conductivity of the dialysis fluid.

5. Apparatus according to one of Claims 1 to 4, **characterized in that** it comprises means (26) for estimating the patient's blood sodium level.

6. Apparatus according to Claims 4 and 5, **characterized in that** the estimator (22) comprises means for optimizing the weight loss rate and the conductivity of the dialysis fluid, which can estimate the coefficients of the model on the basis of an evaluation of the variation in the blood volume as a function of the weight loss rate, of the conductivity of the dialysis fluid and of the blood sodium level.

7. Apparatus according to one of Claims 1 to 6, **characterized in that** it comprises means (36) for detecting a deviation, capable of detecting a possible deviation of the actual patient parameters outside the permitted value intervals.

8. Apparatus according to Claim 7, **characterized in that** the means (36) for detecting the deviation comprise means (37) for observing the deviation and means (38) for signalling the deviation, and **in that** the observer (37) comprises means for calculating the time derivatives of the differences between the actual values and the permitted values of the variation in the blood volume and between the actual values and the permitted values of the weight loss.

9. Apparatus according to Claim 2, **characterized in that** the device for inputting and displaying data comprises means for synthetic representation of simultaneous positions of the patient variables and of the permitted value intervals.

```
                    ┌──────────┐
                    │  DEPART  │
                    └──────────┘
                         │
                         ▼
        ┌─────────────────────────────────┐
        │  MEMORISATION DES VALEURS        │ ─── 1
        │  PERMISES DE BV                  │
        └─────────────────────────────────┘
                         │
                         ▼
        ┌─────────────────────────────────┐
        │  MEMORISATION DES VALEURS        │ ─── 2
        │  PERMISES DE WL                  │
        └─────────────────────────────────┘
                         │
                         ▼
        ┌─────────────────────────────────┐
        │  MEMORISATION DES VALEURS        │ ─── 3
        │  PERMISES DE WLR                 │
        └─────────────────────────────────┘
                         │
                         ▼
        ┌─────────────────────────────────┐
        │  MEMORISATION DES VALEURS        │ ─── 4
        │  PERMISES DE CD                  │
        └─────────────────────────────────┘
                         │
                         ▼
        ┌─────────────────────────────────┐
        │        MESURE DE WL             │ ─── 5
        └─────────────────────────────────┘◄──┐
                         │                     │
                         ▼                     │
        ┌─────────────────────────────────┐   │
        │        MESURE DE BV             │ ── 6│
        └─────────────────────────────────┘   │
                         │                     │
                         ▼                     │
        ┌─────────────────────────────────┐   │
        │  CALCUL DES VALEURS             │ ── 7│
        │  OPERATOIRES DE WLR et CD       │   │
        └─────────────────────────────────┘   │
                         │                     │
                         ▼                     │
        ┌─────────────────────────────────┐   │
        │  CORRECTION DE                  │ ── 8│
        │  WLR et CD                      │   │
        └─────────────────────────────────┘   │
                         │                  9  │
                         ▼                     │
                    ╱──────────╲   NON         │
                   ╱  FIN DE    ╲──────────────┘
                   ╲ TRAITEMENT?╱
                    ╲──────────╱
                         │ OUI
                         ▼
                    ┌──────────┐
                    │   FIN    │
                    └──────────┘
```

Fig.1

11

DEPART

MEMORISATION DE TBV ± ΔTBV — 1a

MEMORISATION T — 1b

CALCUL DES VALEURS PERMISES DE BV — 1c

FIN

Fig.2

DEPART

MEMORISATION DE TWL ± ΔTWL — 2a

MEMORISATION T — 2b

CALCUL DES VALEURS PERMISES DE WL — 2c

FIN

Fig.3

Fig 4

Fig. 5

EP 0 778 783 B1

Fig.6

EP 0 778 783 B1

Fig. 7